# EUROPEAN PATENT APPLICATION

(11) **EP 3 858 272 A1**
(43) Date of publication of application: **04.08.2021**
(21) Application number: 19866060.7
(22) Date of filing: 11.01.2019
(51) Int. Cl.: A61B 17/80

(54) **LATERAL-WING-ENCIRCLING BONE PLATE FOR DISTAL FIBULA TYPE-B FRACTURE**

(30) Priority: 30.09.2018 CN 201811159902
(71) Applicant: Chuang Mei de Medical Device (Tianjin) Co., Ltd., Tianjin 300457 (CN)
(72) Inventor: LIU, Lina, Tianjin 300457 (CN); WANG, TIanxing, Tianjin 300457 (CN); PAN, Binghui, Tianjin 300457 (CN); SHI, Zhongmin, Tianjin 300457 (CN); HONG, Jinsong, Tianjin 300457 (CN); XU, Hailin, Tianjin 300457 (CN)
(74) Representative: Bandpay & Greuter
(86) International application number: PCT/CN2019/071247
(87) International publication number: WO 2020/062709

(57) **Abstract**

It's a distal fibula bone plate with side wing used for type B fracture, comprising a shaft, and a wing. Multiple bearing locking screw holes, locking screw holes, and guide wire holes are set on the shaft. Tabs are provided on both sides of the front part of the shaft, and the rear part is twisted to one direction. The side wing is set protrudingly on one side of the shaft. A sliding compression hole is provided on the side wing. The tabs can be used with instruments to embrace the bones and increase the stability of the bone plate. The bone plate that fits the human fibula has better fit and less impact on skin tension. The bearing locking screw hole on the bone plate allows the locking screw to be fixed in multiple directions, which is convenient to avoid the articular surface, and the cross fixation shall improve the fixing strength. The sliding compression hole on the wing can not only be fixed with screws, but also can be elastically fixed with buttons and nylon threads, increasing the surgeon's choice of surgery.

## Description

### Technical Field

The invention relates to the technical field of orthopedic medical devices, in particular to a distal fibula bone plate with side wing used for type B fracture.

### Background technique

Fracture is one of the most common injuries. As the population ages, the annual incidence of fractures is gradually increasing. Distal fibula fracture is a common fracture. K-wire, tension belt, screws etc. lack firm fixation of the distal end of the fracture. There is almost no specific bone plate for type B fractures, which cannot fully meet the requirements of bone reduction and fixation. Although the ordinary bone plate has a certain fixing strength, the fit cannot be fully satisfied, and complications such as delayed skin healing are prone to occur. The existing anatomical bone plate or ordinary locking bone plate does not have the multi-angle fixation of screws. The screws are prone to loosening during use, which weakens the holding force. The screws of the prior art bone plate are easy to hit the articular surface during use, which causes difficulty in clinical application, affects the treatment effect, and brings pain to patients.

### Invention contents

The purpose of the present invention is to address the above-mentioned shortcomings of the prior art, and provide a distal fibula bone plate with side wing used for type B fracture that meet the characteristics of the distal fibula type B fracture. It can reduce soft tissue stimulation, realize effective compression, get good fit, and get effective fixation.

The technical solution adopted by the present invention to solve the technical problems existing in the known technology is:
The present invention is a distal fibula bone plate with side wing used for type B fracture, comprising a shaft, and a wing. Multiple bearing locking screw holes, locking screw holes, and guide wire holes are set on the shaft. Tabs are provided on both sides of the front part of the shaft, and the rear part is twisted to one direction so as to fit the fibula bone surface. The side wing is set protrudingly on one side of the shaft. A sliding compression hole is provided on the side wing.

The present invention can also be realized with the following measures:
5 bearing locking screw holes, 2 locking screw holes, and 4 guide wire holes are set on the front part of the above mentioned shaft; 4 locking screw holes are set on the rear part of the shaft; a sliding compression hole is set on the side wing.

The sliding compression hole on the above mentioned wing can be fixed with screws, and can also be elastically fixed with buttons and nylon threads.

The shaft and wing are an integral structure.

Bearing locking screw holes on the shaft face different directions.

The advantages and positive effects of the present invention are:
The distal fibula bone plate with side wing used for type B fracture of present invention comprises tabs on its shaft, and a wing. During use, the tabs can be deformed to embrace and fix the bone so as to make the combination between bone plate and fibula more stable. 5 bearing locking screw holes are set on the shaft, which can make the locking screws be fixed in multiple directions, which is convenient to avoid the joint surface, and the cross fixation improves the fixing strength. The rear part of the plate is twisted to one direction. The invention fits the human fibula, which has better fit and less impact on skin tension. The sliding compression hole on the wing can not only be fixed with screws, but also can be elastically fixed with buttons and nylon threads, increasing the surgeon's choice of surgery.

### Figures

Figure 1 is a side view of the distal fibula bone plate with side wing used for type B fracture of present invention.
Figure 2 is a front view of the distal fibula bone plate with side wing used for type B fracture of present invention.

### Detailed description of the present invention

The following contents are detailed description of the present invention.

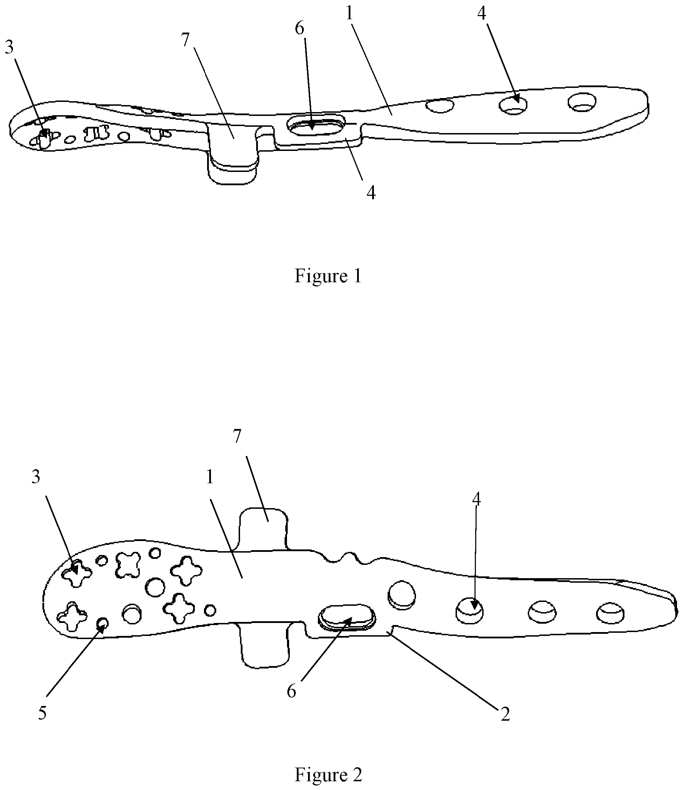

As shown in Figure 1 to figure 2, the distal fibula bone plate with side wing used for type B fracture of present invention comprises a shaft 1, and a side wing 2. Five bearing locking screw holes 3, six locking screw holes 4, and four guide wire holes 5, are set on the shaft. For locking screw holes, two are set on the front part of the shaft while four are set on the rear part of the shaft. The side wing is set protrudingly on one side of the shaft. The shaft and wing are an integral structure. A sliding compression hole 6 is provided on the side wing 2. The tabs 7 are set on both sides of the front part of the shaft, which can be used with instruments to embrace the bones and increase the stability of the bone plate. The rear part of the shaft is twisted to one direction so as to fit the fibula bone and has less impact on skin tension. The bearing locking screw hole on the shaft allows the locking screw to be fixed in multiple directions, which is convenient to avoid the articular surface, and the cross fixation shall improve the fixing strength. The sliding compression hole on the wing can not only be fixed with screws, but also can be elastically fixed with buttons and nylon threads, increasing the surgeon's choice of surgery.

When the present invention is used, the bearing locking screw hole on the bone plate is matched with the bearing locking screw, in order to fix the bone plate and the bone. The bearing locking screw can be matched with the bearing locking screw hole within the range of the angle ±15° to avoid the joint surface and prevent additional trauma to the patient. During the operation, determine the fixed position and quantity of the bearing locking screw hole and the locking screw hole according to the actual situation. The structure and usage of the bearing locking screw hole and the bearing locking screw belong to the prior art in this field, and will not be repeated here.

The setting directions of the bearing locking screw holes of the shaft face different directions, which further expand the setting angle and range of the bearing locking screw in use.

The above are only the preferred embodiments of the present invention, and do not limit the present invention in any form. Although the present invention has been disclosed as above in preferred embodiments, it is not intended to limit the present invention. Any person familiar with the profession, without departing from the scope of the technical solution of the present invention, will of course use the disclosed technical content to make some changes or modifications to become equivalent embodiments with equivalent changes. However, without departing from the content of the technical solution of the present invention, any simple modifications, equivalent changes and modifications made to the above embodiments based on the technical essence of the present invention shall fall within the scope of the technical solution of the present invention.

## Claims

1. It's a distal fibula bone plate with side wing used for type B fracture. The features lie in: it comprises a shaft, and a wing. Multiple bearing locking screw holes, locking screw holes, and guide wire holes are set on the shaft. Tabs are provided on both sides of the front part of the shaft, and the rear part is twisted to one direction so as to fit the fibula bone. The side wing is set protrudingly on one side of the shaft. A sliding compression hole is provided on the side wing.

2. According to claim 1, the above distal fibula bone plate with side wing used for type B fracture, its features lie in: 5 bearing locking screw holes, 2 locking screw holes, and 4 guide wire holes are set on the front part of the shaft; 4 locking screw holes are set on the rear part of the shaft; a sliding compression hole is set on the wing.

3. According to claim 1 or 2, the above distal fibula bone plate with side wing used for type B fracture, its features lie in: The sliding compression hole on the wing can be fixed with screws, or can also be elastically fixed with buttons and nylon threads.

4. According to claim 3, the above distal fibula bone plate with side wing used for type B fracture, its features lie in: the shaft and wing are an integral structure.

5. According to claim 1, the above distal fibula bone plate with side wing used for type B fracture, its features lie in: bearing locking screw holes on the shaft face different directions.
